# EUROPEAN PATENT APPLICATION

(11) **EP 0 616 800 A2**
(43) Date of publication of application: **28.09.1994**
(21) Application number: 94102193.3
(22) Date of filing: 12.02.1994
(51) Int. Cl.: A61K 7/46, A61K 7/32, A61K 7/48

(54) **Odorant compositions with prolonged diffusion**

(30) Priority: 22.03.1993 EP 93810205
(71) Applicant: GIVAUDAN-ROURE (INTERNATIONAL) S.A., CH-1214 Vernier (CH); F. HOFFMANN-LA ROCHE AG, CH-4002 Basel (CH)
(72) Inventor: Sauvage, Françoise, F-92270 Bois-Colombes (FR); Blakeway, John Murray, F-95420 Nucourt (FR)
(74) Representative: Urech, Peter, Dr.

(57) **Abstract**

Novel odorant compositions comprising ethanol, perfume concentrate and panthenol ethyl ether.

## Description

The present invention is concerned with novel odorant compositions. More particularly, the invention is concerned with novel odorant compositions with prolonged diffusion after the application on the skin.

It is well known that mixtures of perfume materials when deposited on the skin lose intensity and may change character with time, mainly due to factors such as differential evaporation and skin penetration.

Many attempts have been made to minimize these drawbacks, but so far without notable success. Particularly, efforts have been made to prolong the diffusion, as well as to improve other characteristics of perfume materials, by e.g. increasing the perfume concentrate concentration or by using additives such as silicones, glycerol, polyethylene glycols and so on. Such perfume compositions, however, have never been really successful as the results obtained were only marginal.

It thus actually existed a problem in this connection, which now has been overcome by the present invention.

Surprisingly, it has now been found that the addition of panthenol ethyl ether, i.e. the compound of formula 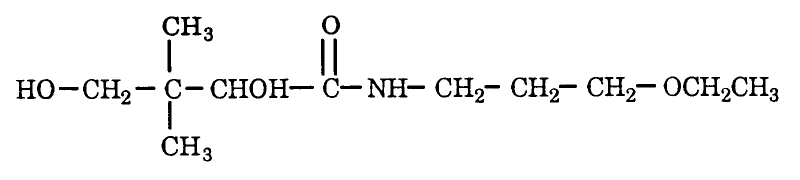
significantly improves and particularly prolongs diffusion of perfume materials from the skin, without notably modifying the olfactive note of the product. The present invention thus concerns odorant compositions comprising from about 70, conveniently from about 75, or even from about 80 to about 99% of ethanol, from about 0.5 to about 30% of perfume concentrate, from about 0.1 to about 20% of panthenol ethyl ether and from about 0% to about 30% of water.

The term "odorant composition", as used in connection with this invention, means solutions of perfume materials in alcohol and stands for well known commercial products such as e.g. Deo-Cologne, Eau de Cologne, Eau de Toilette, Perfume Extrait and so on.

The term "perfume concentrate" stands for the required mixture of perfume materials of synthetic and/or natural origin.

The term "panthenol" as used in connection with the present invention stands for DL-panthenol or for D-panthenol.

The percentages referred to in the scope of the present invention are on a weight by weight basis.

The amount of perfume concentrate and thus also the amount of ethanol present depends upon the respective odorant composition and can be easily determined by the skilled artisan. The amount of panthenol ethyl ether can also vary within a range of from about 0.1 to about 20%, but lies preferably in the range of from about 0.5 to about 5%. By way of examples, in the following odorant compositions the respective ingredients may be present in the following approximate amounts:

| a) Deo-Cologne: | |
|---|---|
| Perfume concentrate | 0.5-3% |
| Panthenol ethyl ether | 0.5-2% |
| Ethanol (98°) | q.s. |

| b) Eau de Cologne: | |
|---|---|
| Perfume concentrate | 2-7% |
| Panthenol ethyl ether | 0.5-3% |
| Ethanol (88° or 73°) | q.s. |

| c) Eau de Toilette: | |
|---|---|
| Perfume concentrate | 5-20% |
| Panthenol ethyl ether | 0.5-5% |
| Ethanol (93°) | q.s. |

| d) Perfume Extrait: | |
|---|---|
| Perfume concentrate | 15-25% |
| Panthenol ethyl ether | 0.5-5% |
| Ethanol (96°) | q.s. |

With the alcohol also water is introduced into the compositions, and the degrees of alcohol mean % v/v at 20°C.

Besides the ingredients mentioned before, the odorant compositions according to the present invention can also contain additives which are well known in the art. By way of example the following can be mentioned: colorants, UV-absorbants, antioxydants, preservatives, emollients, natural herbal extracts, germicides, deodorants and so on. If such additions are present their amount lies preferably between about 0.05 and about 2%. The same applies to the cosmetic additive phytantriol, i.e. the compound of formula 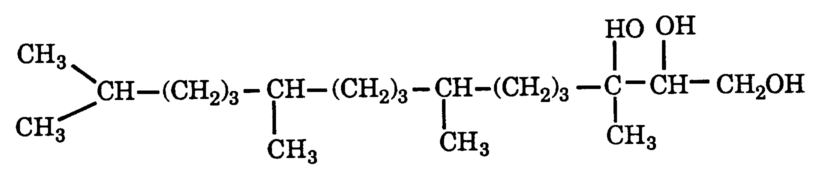
which compound, can, in some cases, even improve or reinforce the present effect of pantenol ethyl ether. In such compositions, phytantriol can thus replace part of the panthenol ethyl ether, say about 10 to about 80% of this ether.

Furthermore, the odorant compositions can also contain vitamins, such as e.g. vitamin E, preferably in the form of the acetate, vitamin A, preferably in the form the acetate or any other common ester, vitamin C, preferably in the form of the palmitate, and so on.

The following examples are illustrative of the present invention and are by no means intended to limit the scope of the present invention.

### Example 1

In a manner known per se a Deo-Cologne of the following composition has been prepared:

| | |
|---|---|
| Perfume concentrate | 1% |
| D-Panthenol ethyl ether | 1% |
| Ethanol (96°) | 98% |

### Example 2

In a manner known per se an Eau de Cologne of the following composition has been prepared:

| | |
|---|---|
| Perfume concentrate | 5% |
| D-Panthenol ethyl ether | 2% |
| Ethanol (90°) | 93% |

### Example 3

In a manner known per se an Eau de Toilette of the following composition has been prepared:

| | |
|---|---|
| Perfume concentrate | 10% |
| D-Panthenol ethyl ether | 2% |
| Ethanol (96°) | 88% |

## Claims

1. Odorant composition comprising from about 70 to about 99% of ethanol, from about 0.5 to about 30% of perfume concentrate, from about 0.1 to about 20% of panthenol ethyl ether and from about 0% to about 30% of water.

2. Odorant composition according to Claim 1, in the form of a Deo-Cologne, comprising from about 0.5 to about 3% of perfume concentrate and from about 0.5 to about 2% of panthenol ethyl ether.

3. Odorant composition according to Claim 1, in the form of an Eau de Cologne, comprising from about 2 to about 7% of perfume concentrate and from about 0.5 to about 3% of panthenol ethyl ether.

4. Odorant composition according to Claim 1, in the form of an Eau de Toilette, comprising from about 5 to about 20% of perfume concentrate and from about 0.5 to about 5% of panthenol ethyl ether

5. Odorant composition according to Claim 1, in the form of a Perfume Extrait, comprising from about 15 to about 25% of perfume concentrate and from about 0.5 to about 5% of panthenol ethyl ether.

6. An odorant composition according to any one of Claims 1 to 5, in the form of a solution and intended for improving and/or prolonging diffusion of the perfume materials after the application on the skin.

7. The use of panthenol ethyl ether in odorant compositions containing ethanol for improving/or prolonging diffusion after the application on the skin.

8. Method of improving and/or prolonging diffusion of perfume materials from the skin, comprising incorporating panthenol ethyl ether into an odorant composition containing ethanol.
